(19) Europäisches Patentamt
European Patent Office
Office européen des brevets

(11) Veröffentlichungsnummer: **0 167 045**
**A1**

(12) **EUROPÄISCHE PATENTANMELDUNG**

(21) Anmeldenummer: 85107403.9

(22) Anmeldetag: 15.06.85

(51) Int. Cl.⁴: C 07 D 471/04
A 61 K 31/435
//(C07D471/04, 221:00, 221:00)

(30) Priorität: 22.06.84 DE 3423003

(43) Veröffentlichungstag der Anmeldung: 08.01.86 Patentblatt 86/2

(84) Benannte Vertragsstaaten: AT BE CH DE FR GB IT LI NL SE

(71) Anmelder: Beiersdorf Aktiengesellschaft
Unnastrasse 48
D-2000 Hamburg 20(DE)

(72) Erfinder: Cohnen, Erich, Dr.
Moorende 74
D-2155 Jork(DE)

(72) Erfinder: Hofferber, Eva, Dr.
Emilienstrasse 47
D-2000 Hamburg 20(DE)

(72) Erfinder: Beuttler, Thomas, Dr.
Hahnenfussweg 6
D-2000 Hamburg 55(DE)

(54) **Benzo (c)(1,8)naphthyridine, Verfahren zu ihrer Herstellung und ihre Verwendung sowie diese Verbindungen enthaltende Zubereitungen.**

(57) Benzo[c][1,8]naphthyridine der allgemeinen Formel I,

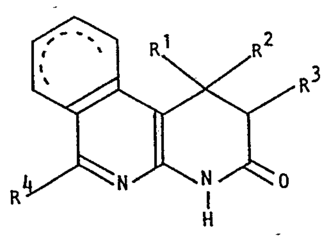

(I)

worin

$R^1$ Wasserstoff, eine Alkylgruppe mit 1 bis 6 Kohlenstoffatomen, die Phenylgruppe oder eine substituierte Phenylgruppe, eine Cycloalkylgruppe, eine Carboxylgruppe, Alkoxycarbonylgruppe, Carboxamidgruppe oder Cyanogruppe,

$R^2$ Wasserstoff oder eine Alkylgruppe mit 1 bis 4 Kohlenstoffatomen,

$R^3$ Wasserstoff, eine Alkylgruppe mit 1 bis 4 Kohlenstoffatomen, eine Carboxylgruppe, Alkoxycarbonyl-gruppe, Carboxamidgruppe oder Cyanogruppe und

$R^4$ Wasserstoff, Halogen, die Cyanogruppe, Hydroxylgruppe, Carboxamidgruppe, eine Alkylthiogruppe oder Alkoxygruppe mit jeweils 1 bis 6 Kohlenstoffatomen, die gegebenenfalls durch substituierte Aminogruppen substituiert sein können, eine Aminogruppe oder substituierte Aminogruppe, eine Pyrrolidino-, Piperidino-, Hydrazino-, Morpholino-, Piperazino- oder substituierte Piperazinogruppe, bedeutet, wobei, wie durch punktierte Bindungen angegeben, Ring C des Benzonaphthyridingerüstes auch in partiell hydrierter Form vorliegen kann und Alkyle jeweils geradkettig oder verzweigt sein können, sowie ihre tautomeren Formen und ihre Salze sowie Säureadditionssalze, besitzen positiv inotrope und vasodilatierende Eigenschaften und eignen sich als Arzneimittel zur Behandlung von Herzinsuffizienz, Angina pectoris und/oder Hypertonie.

EP 0 167 045 A1

- 1 -

Beiersdorf Aktiengesellschaft
Hamburg

# Benzo/c7/1,87naphthyridine, Verfahren zu ihrer Herstellung und ihre Verwendung sowie diese Verbindungen enthaltende Zubereitungen

Gegenstand der Erfindung sind Benzo/c7/1,87-naphthyridine der allgemeinen Formel I,

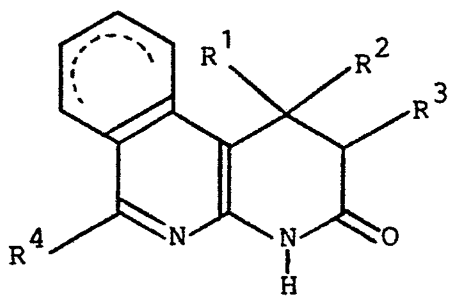

(I)

worin $R^1$ Wasserstoff, eine Alkylgruppe mit 1 bis 6 Kohlenstoffatomen, die Phenylgruppe oder eine substituierte Phenylgruppe, eine Cycloalkylgruppe, eine Carboxylgruppe, Alkoxycarbonylgruppe, Carboxamidgruppe oder Cyanogruppe,

$R^2$ Wasserstoff oder eine Alkylgruppe mit 1 bis 4 Kohlenstoffatomen,

$R^3$ Wasserstoff, eine Alkylgruppe mit 1 bis 4 Kohlenstoffatomen, eine Carboxylgruppe, Alkoxycarbonyl-

gruppe, Carboxamidgruppe oder Cyanogruppe und
$R^4$ Wasserstoff, Halogen, die Cyanogruppe, Hydroxylgruppe, Carboxamidgruppe, eine Alkylthiogruppe oder Alkoxygruppe mit jeweils 1 bis 6 Kohlenstoffatomen, die gegebenenfalls durch substituierte Aminogruppen substituiert sein können, eine Aminogruppe oder substituierte Aminogruppe, eine Pyrrolidino-, Piperidino-, Hydrazino-, Morpholino-, Piperazino- oder substituierte Piperazinogruppe,

bedeutet, wobei, wie durch punktierte Bindungen angegeben, Ring C des Benzonaphthyridingerüstes auch in partiell hydrierter Form vorliegen kann und Alkyle jeweils geradkettig oder verzweigt sein können, sowie ihre tautomeren Formen und ihre Salze sowie Säureadditionssalze, Verfahren zu ihrer Herstellung und ihre Verwendung und Zubereitungen, die diese Verbindungen enthalten.

Die Verbindungen 1,2-Dihydro-benzo[c][1,8]-naphthyridin-3(4H)-on und 6-Brom-1,2-dihydro-benzo-[c][1,8]-naphthyridin-3(4H)-on sind in der Literatur ohne Wirkungsangabe genannt (F. Johnson und W.A. Nasutavicus, J. Org. Chem. 27, 3953 (1962)). Sie besitzen aber die Eigenschaften der übrigen erfindungsgemäßen Verbindungen. Alle Angaben, die für die übrigen erfindungsgemäßen Verbindungen gemacht werden, gelten auch für diese Verbindungen. Die Verbindungen der Formel I, ausgenommen diese beiden vorstehend genannten Verbindungen, sind neu.

Der Einfachheit halber sind die erfindungsgemäßen Verbindungen in nur einer durch Formel I wiedergegebenen tautomeren Form definiert. Die Erfindung erstreckt sich jedoch auf alle tautomeren Formen der Verbindungen.

Obgleich pharmazeutisch verträgliche Salze und Säureadditionssalze der Verbindungen der Formel I bevorzugt sind, liegen alle Salze innerhalb des Bereichs der Erfindung. Alle Salze sind wertvoll zur Herstellung der Basen, selbst wenn das spezielle Salz nur als Zwischenprodukt gewünscht wird, wie zum Beispiel, wenn das Salz nur für Zwecke der Reinigung oder Identifizierung gebildet wird, oder wenn es als ein Zwischenprodukt bei der Herstellung eines pharmazeutisch verträglichen Salzes, wie beispielsweise durch Ionenaustauschverfahrensweisen, verwendet wird.

Die Verbindungen der allgemeinen Formel I und deren Salze enthalten asymmetrische Kohlenstoffatome. Daher sind auch die verschiedenen optischen Isomeren sowie die Diastereoisomeren Gegenstand der Erfindung ebenso wie die Salze und Additionssalze dieser Verbindungen mit Säuren. Racemate können nach an sich bekannten Methoden in ihre optischen Antipoden aufgetrennt werden.

Bevorzugt werden Verbindungen der Formel I mit aromatischem C-Ring, in der mindestens einer der Substituenten $R^1$, $R^2$, $R^3$ oder $R^4$ einen Rest bedeutet, der nicht Wasserstoff ist, wobei, wenn $R^4$ Brom ist, auch mindestens einer der Substituenten $R^1$, $R^2$ oder $R^3$ einen Rest bedeutet, der nicht Wasserstoff ist. Ebenfalls werden die Verbindungen der Formel I bevorzugt, in denen der C-Ring des Benzonaphthyridingerüstes partiell hydriert ist.

Die erfindungsgemäßen Alkylgruppen sowie die Alkylteile der Ester und Alkoxygruppen können geradkettig oder verzweigt sind und sind vorzugsweise Methyl-, Ethyl- und Propylgruppen.

Halogen ist Fluor, Chlor, Brom oder Jod, vorzugsweise Brom.

Die Substituenten $R^1$ und/oder $R^2$ sind vorzugsweise Alkyl, insbesondere Methyl und Ethyl.

Bevorzugte Cycloalkylgruppen sind die Cyclopropyl-, Cyclobutyl-, Cyclohexyl- und die Cyclopentylgruppe.

Alkoxycarbonylgruppen $R^1$ und $R^3$ sind vorzugsweise niedere Estergruppen, insbesondere Methoxy-, Ethoxy- und Propoxycarbonylgruppen.

Die Substituenten $R^2$ und $R^3$ sind vorzugsweise Wasserstoff.

$R^2$ ist vorzugsweise dann Wasserstoff, wenn $R^1$ einen anderen Rest als Wasserstoff bedeutet.

Bevorzugte Reste $R^4$ sind Halogen, vorzugsweise Brom, Cyano-, Hydroxy-, Alkoxy-, insbesondere Methoxy- und Ethoxygruppen und insbesondere Amino-und substituierte Aminogruppen.

Bevorzugte substituierte Aminogruppen, die Substituenten der Alkylthio- oder Alkoxygruppen bilden, sind mono- oder dialkylierte Aminogruppen mit bis zu 6, vorzugsweise bis zu 3 Kohlenstoffatomen je Alkylrest.

Substituierte Aminogruppen $R^4$ sind mono- oder vorzugsweise disubstituierte Aminogruppen, insbesondere Alkylaminogruppen. Diese Alkylgruppen können geradkettig oder verzweigt sein und enthalten 1 bis 6, vorzugsweise 1 bis 3 Kohlenstoffatome und sie können ebenfalls substituiert sein. Bevorzugte Substituenten der Alkylgruppen sind Arylgruppen, insbesondere die Phenylgruppe. Besonders bevorzugte Substituenten der Aminogruppe sind Arylalkylgruppen, vorzugsweise Benzylgruppen.

Weitere bevorzugte substituierte Aminogruppen $R^4$ sind vorzugsweise in 4-Stellung substituierte oder unsubstituierte Piperazingruppen und die Hydrazinogruppe. Vorzugsweise trägt die Piperazingruppe eine Alkoxycarbonylgruppe, insbesondere Methoxy-, Ethoxy- oder Propoxycarbonylgruppen oder eine Benzylgruppe.

Besonders bevorzugte substituierte Aminogruppen $R^4$ sind mono- oder disubstituierte Aminogruppen-$NR^5R^6$, worin $R^5$ und $R^6$, die gleich oder verschieden sein können, Wasserstoff, Arylalkylgruppen, verzweigte oder unverzweigte Alkylgruppen mit jeweils 1 bis 6 Kohlenstoffatomen sind, die gegebenenfalls durch Alkoxy-, Aryloxy- oder substituierte Aryloxygruppen, Pyridinylgruppen, Cycloalkylgruppen oder substituierte Aminogruppen substituiert sein können.

Vorzugsweise sind die Alkoxysubstituenten der Alkylgruppe $R^5$ und $R^6$ geradkettig oder verzweigt und besitzen insbesondere 1 bis 6, bevorzugt 1 bis 3 Kohlenstoffatome. Aryloxy ist vorzugsweise Phenoxy, bevorzugte Substituenten dafür sind geradkettige oder verzweigte Alkoxygruppen, insbesondere mit 1 bis 6, vorzugsweise 1 bis 3 Kohlenstoffatomen. Besonders bevorzugt ist die 2-Methoxyphenoxygruppe.

Substituierte Aminogruppen, die Substituenten der Alkylgruppen $R^5$ und $R^6$ bilden, sind vorzugsweise mono- oder dialkylierte Aminogruppen mit jeweils 1 bis 6, insbesondere jeweils 1 bis 3 Kohlenstoffatomen, die Piperazino- oder eine substituierte Piperazinogruppe. Bevorzugte Piperazino-Substituenten sind Aryl-, insbesondere Alkoxyphenylgruppen. Vorzugsweise ist die Piperazinogruppe in 1,4-Stellung substituiert. Besonders bevorzugt ist die 2-Methoxyphenylpiperazinogruppe.

Im C-Ring des Benzonaphthyridingerüstes können die 7-, 8-, 9- und 10-Positionen vollständig, partiell oder nicht hydriert sein, wie in Formel I durch die punktierten Bindungen angegeben, so daß Doppelbindungen in 7,8- und/oder 9,10- sowie 8,9-Position, vorliegen können. Bevorzugt sind davon die nicht hydrierten, aromatischen Verbindungen sowie die 7,8,9,10-Tetrahydro-Verbindungen.

Neben den in den Beispielen genannten Verbindungen werden die folgenden erfindungsgemäßen Verbindungen und ihre Salze bevorzugt:

1-Methyl-6-brom-1,2-dihydro-benzo[c][1,8]naphthyridin-3(4H)-on

1-Methyl-1,2-dihydro-benzo[c][1,8]naphthyridin-3(4H)-on

1-Methyl-6-methoxy-1,2-dihydro-benzo[c][1,8]-naphthyridin-3(4H)-on

1-Methyl-6-propoxy-1,2-dihydro-benzo[c][1,8]-naphthyridin-3(4H)-on

1-Methyl-6-dimethylamino-1,2-dihydro-benzo[c][1,8]-naphthyridin-3(4H)-on

1-Methyl-6-diethylamino-1,2-dihydro-benzo[c][1,8] naphthyridin-3(4H)-on

1-Cyano-6-amino-1,2-dihydro-benzo[c][1,8]naphthyridin-3(4H)-on

1-Methyl-1,2,7,8,9,10-hexahydro-benzo[c][1,8]naphthyridin-3(4H)-on

1-Methyl-6-diethylamino-1,2,7,8,9,10-hexahydro-benzo-[c][1,8]naphthyridin-3(4H)-on

1-Ethyl-6-brom-1,2-dihydro-benzo[c][1,8]naphthyridin-3(4H)-on

1-Methyl-6-di-n-propylamino-1,2-dihydro-benzo-[c][1,8]naphthyridin-3(4H)-on

1-Methyl-6-n-butylamino-1,2-dihydro-benzo[c][1,8]-naphthyridin-3(4H)-on

1-Methoxycarbonyl-6-bromo-1,2-dihydro-benzo[c][1,8]-naphthyridin-3(4H)-on

1-Ethoxycarbonyl-6-diethylamino-1,2-dihydro-benzo-[c][1,8]naphthyridin-3(4H)-on

1-Cyano-6-diethylamino-1,2-dihydro-benzo[c][1,8]-naphthyridin-3(4H)-on

1-Cyano-6-brom-1,2-dihydro-benzo[c][1,8]naphthyridin-3(4H)-on

1-Cyano-6-ethoxy-1,2-dihydro-benzo[c][1,8]naphthyridin-3(4H)-on

1-Methyl-6-hydrazino-1,2-dihydro-benzo[c][1,8]-naphthyridin-3(4H)-on

1-Methyl-6-amino-1,2-dihydro-benzo[c][1,8]naphthyridin-3(4H)-on

Die folgenden Verbindungen der allgemeinen Formel I und deren Salze mit hohem therapeutischen Effekt werden besonders bevorzugt, und zwar in der Form der Racemate sowie in der Form optisch aktiver Isomerer:

a) 1-Methyl-6-dimethylamino-1,2-dihydro-benzo/c7/1,87-naphthyridin-3(4H)-on,

b) 1-Methyl-6-diethylamino-1,2-dihydro-benzo/c7/1,87-naphthyridin-3(4H)-on,

c) 1-Methyl-6-di-n-propylamino-1,2-dihydro-benzo-/c7/1,87naphthyridin-3-(4H)-on.

d) 1-Methyl-6-methylamino-1,2-dihydro-benzo/c7/1,87 naphthyridin-3(4H)-on

e) 1-Methyl-6-cyano-1,2 -dihydro-benzo/c7/1,87naphthyridin-3(4H)-on

f) 1-Methyl-6-isopropylamino-1,2-dihydro-benzo/c7/1,87 naphthyridin-3(4H)-on

g) 1-Methyl-6-{2-/4-(2-methoxy-phenyl)-1-piperazinyl7-ethylamino}-1,2-dihydro-benzo/c7/1,87naphthyridin-3(4H)-on

h) 1-Methyl-6-{3-/4-(2-methoxy-phenyl)-1-piperazinyl7-propylamino}-1,2-dihydro-benzo/c7/1,87naphthyridin-3(4H)-on

Die erfindungsgemäßen Verbindungen der Formel I sowie deren Isomere, ihre physiologisch verträglichen Salze und Säureadditionssalze sind therapeutische Wirkstoffe, besitzen hohe pharmakologische Wirkung und sind wertvolle Arzneimittel. Insbesondere zeigen sie cardiovasculäre Wirkungen. Sie besitzen positiv inotrope und vasodilatierende Eigenschaften und eignen sich deshalb zur Behandlung der Herzinsuffizienz, insbesondere der chronischen Herzinsuffizienz, der Angina pectoris und/oder der Hypertonie.

Nach i.v. bzw. i.d. Applikation in Dosierungen von 0,3 mg-3,0 mg/kg bzw. 1-10 mg/kg kommt es bei Katze bzw. Schwein zu einer länger anhaltenden blutdrucksenkenden Wirkung, die von einem anhaltenden positiv inotropen Effekt begleitet ist, der sich auch in vitro am Papillarmuskel oder Atrium des Meerschweinchens eindrucksvoll nachweisen läßt.

Die Verbindungen der vorliegenden Erfindung können oral oder parenteral angewendet werden. Die Einzeldosierungen für die orale Anwendung liegen beim Menschen bei 1-100mg, vorzugsweise 5-50 mg, insbesondere 20-30 mg. Diese Dosierungen sind vorteilhaft zur Behandlung der vorstehend genannten Krankheiten, insbesondere der Herzinsuffizienz.

Gemäß der Erfindung werden pharmazeutische Zusammensetzungen geschaffen, die eine Verbindung der Formel I oder deren pharmazeutisch verträgliche Salze zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups, Suspensionen und Flüssigkeiten oder parenteral in Form von Lösungen oder Suspensionen verabreicht werden. Oral zu verabreichende Präparate können einen oder mehrere Zusätze wie Süßungsmittel, Aromatisierungsmittel, Farbstoffe und Konservierungsmittel enthalten. Tabletten können den Wirkstoff mit üblichen pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln

und Mitteln, die den Zerfall der Tabletten bei oraler Verabreichung fördern wie Stärke oder Alginsäure, Bindemitteln wie Stärke, oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Ethanol, Propylenglycol, Ether des Tetrahydrofurfurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyoxyethylenstearat und Polyoxyethylensorbitanmonooleat, und Konservierungsmitteln wie Ethylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90% , insbesondere 1 bis 90%, enthalten, wobei der Rest ein Trägerstoff oder Zusatzstoff ist. Im Hinblick auf die Herstellung und Verabreichung werden festen Präparate wie Tabletten und Kapseln bevorzugt. Vorzugsweise enthalten die Präparate den Wirkstoff in einer Menge von 5-15 mg.

Die Verbindungen der Formel I werden dadurch hergestellt, daß man eine Verbindung der allgemeinen Formel II

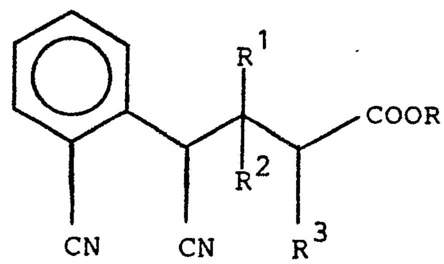

(II)

worin $R^1$, $R^2$ und $R^3$ die angegebene Bedeutung haben und R eine niedere Alkylgruppe - bevorzugt Methyl oder Äthyl - bedeutet, mit einer Halogenwasserstoffsäure insbesondere Bromwasserstoffsäure in an sich bekannter Weise zu Verbindungen der Formel I cyclisiert, wobei $R^4$ ein Halogenatom bedeutet (F. JOHNSON u. W.A. NASUTAVICUS, J. Org. Chem. 27, 3953 (1962).

Die Cyclisierung erfolgt bevorzugt mit Bromwasserstoffsäure in Äther als Lösungsmittel.

Die Verbindungen der allgemeinen Formel I, in der $R^4$ die übrigen angegebenen Bedeutungen besitzt, sind aus den Verbindungen der Formel I mit $R^4$ = Halogen zugänglich.

Durch Hydrierung mit Wasserstoff in Gegenwart eines Katalysators, insbesondere eines Palladium-Aktivkohle-Katalysators, gegebenenfalls unter Zusatz von Natriumacetat, werden die Verbindungen der

Formel I erhalten, in der $R^4$ Wasserstoff bedeutet. Mit Halogeniden oder Cyaniden, insbesondere mit Alkalihalogeniden oder Alkalicyaniden, insbesondere Kaliumcyanid, lassen sich andere Halogenreste oder die Cyanogruppe in an sich bekannter Weise einführen. Mit Basen, insbesondere Alkalihydroxiden, läßt sich die Hydroxygruppe einführen. Vorzugsweise werden die vorstehend genannten Austauschreaktionen in einem Lösungsmittel bei erhöhter Temperatur durchgeführt. Durch partielle Verseifung erhält man aus der Cyanogruppe die Carboxamidgruppe.

Vorzugsweise werden die Verbindungen der Formel I, in denen $R^1$ oder $R^3$ eine Carboxyl-, Carboxamid- oder Cyano-Gruppe symbolisieren, nach literaturbekannten Methoden aus den entsprechenden Carbonsäureestern hergestellt. Durch Umesterung sind in an sich bekannter Weise auch andere Ester erhältlich und durch Spaltung der Ester die freien Carbonsäuren. Säureamide werden aus den Estern durch Umsetzung mit Ammoniak erhalten und die Cyanogruppen enthaltenden Verbindungen durch Dehydratisierung der Amide.

Die in Ring C (Positionen 7, 8, 9, 10) partiell hydrierten Verbindungen der allgemeinen Formel I werden aus den entsprechenden aromatischen Verbindungen der Formel I durch Reduktion mit Wasserstoff in Gegenwart eines Rhodium-Katalysators bei 50 - 100 bar synthetisiert.

Zur Einführung der Alkoxygruppen erhitzt man die Halogen-Verbindung mit Natrium- oder Kaliumalkoxylat in dem entsprechenden Alkohol. Analog werden die Alkylthioverbindungen aus den entsprechenden Mercaptanen ($R^4$—H) erhalten.

In der 6-Stellung substituierte Aminogruppen tragende oder 6-Amino-substituierte Verbindungen der

Formel I erhält man durch Substitution von $R^4$=Halogen, durch ein obiger Definition der Aminoreste entsprechendes Amin (der Formel H-$R^4$) oder Ammoniak in alkoholischer Lösung bei Temperaturen oberhalb 100°C, gegebenenfalls unter Druck. Eingeschlossen sind hier die heterocyclischen Amine und Hydrazin.

Die Ausgangsverbindungen der Formel II können durch Michael-Addition von 2-Cyano-benzylcyanid an Propensäureester der allgemeinen Formel III

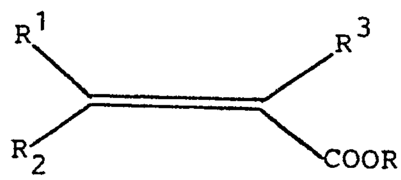

(III)

worin $R^1$, $R^2$, $R^3$ und R die angegebene Bedeutung haben, hergestellt werden.

Die Propensäureester der Formel III sind bekannt oder nach bekannten Methoden erhältlich.

Die Verbindungen der allgemeinen Formel I können sowohl Basen als auch Säuren, beziehungsweise amphoter sein und daher in der Form ihrer Salze oder Säureadditionssalze aus den Reaktionsgemischen isoliert werden. Sie lassen sich als Basen mit geeigneten anorganischen oder organischen Säuren nach bekannten Verfahren in Salze überführen oder bilden als Säuren mit Basen Salze.

Bevorzugt werden physiologisch verträgliche Salze oder Säureadditionssalze. Hierfür sind als anorganische Säuren beispielsweise Halogenwasserstoffsäuren, zum Beispiel Salzsäure oder Schwefelsäure, und als organische Säuren zum Beispiel Fumarsäure, Maleinsäure, Zitronensäure und Weinsäure geeignet. Zur Herstellung wird die heiße alkoholische Lösung der Base mit der alkoholischen Lösung einer geeigneten Säure versetzt und man erhält nach Etherzusatz das Salz. Bevorzugte Salze sind die Alkali, Erdalkali und Ammoniumsalze der Verbindungen der Formel I, die mit den entsprechenden Basen, insbesondere Natrium-, Kalium- oder Ammoniumhydroxid erhalten werden.

Diastereoisomere können in bekannter Weise aufgrund der physikalisch -chemischen Unterschiede ihrer Bestandteile in ihre racemischen Modifikationen getrennt werden. Racemate können nach bekannten Methoden getrennt werden, beispielsweise durch Umkristallisieren in optisch aktiven Lösungsmitteln, durch Mikroorganismen oder Reaktion mit einer optisch aktiven Säure oder Base, die mit der racemischen Verbindung ein Salz bilden, Trennung der Diastereoisomeren durch fraktionierte Kristallisation und Freisetzung der Enantiomeren durch geeignete Mittel. Besonders geeignete optisch aktive Säuren sind beispielsweise die d- und l-Formen der Weinsäure, Ditoluoylweinsäure, Äpfelsäure, Mandelsäure, Kamphersulfonsäure oder Pyrrolidon-carbonsäure. Geeignete optisch aktive Basen sind $\alpha$-Phenyläthylamin, Menthylamin, Ephedrin, Brucin und Chinin. Vorteilhafterweise wird der aktivere der Antipoden isoliert. Gemäß der Erfindung ist es jedoch auch möglich, die reinen Enantiomeren durch asymmetrische Synthese zu erhalten.

Die folgenden Beispiele erläutern die Erfindung.

Beispiel 1

1-Methyl-6-brom-1,2-dihydro-benzo/c//1,8/-naphthyridin-3(4H)-on

Ein Gemisch von 7,1 g (0,05 Mol) 2-Cyano-benzylcyanid und 5,0 g (0,05 Mol) Crotonsäuremethylester wird mit einer katalytischen Menge einer 5%igen methanolischen Lösung von Natriummethylat versetzt und nach Abklingen der Wärmetönung mit Essigsäure neutralisiert. Man nimmt das Reaktionsgemisch in Methylenchlorid auf, wäscht mit Wasser bis zur neutralen Reaktion und dampft nach Trocknen das Lösungsmittel ab. Man erhält als öligen Rückstand 3-Methyl-4-cyano-4-(2-cyano-phenyl)-buttersäure-methylester (Formel II), der ohne weitere Reinigung in 100 ml absolutem Diäthyläther gelöst und durch 5-stündiges Einleiten von HBr cyclisiert wird. Man saugt den gelben Niederschlag ab, digeriert ihn in Natriumhydrogencarbonat-Lösung und kristallisiert aus Ethanol um. Man erhält 4,9 g 1-Methyl-6-brom-1,2-dihydro-benzo/c//1,8/naphthyridin -3(4H)-on.
Fp. 190 - 191°C.

Analog Beispiel 1 wurden folgende Benzo-1,2-dihydro-naphthyridin-Verbindungen der Formel I (aromatischer C-Ring) hergestellt:

| Beispiel Nr. | $R^1$ | $R^2$ | $R^3$ | $R^4$ | Fp. °C |
|---|---|---|---|---|---|
| 2 | H | H | $CH_3$ | Br | 188 |
| 3 | $C_6H_5$ | H | H | Br | 218 |
| 4 | H | H | COOEt | Br | 198-199 |
| 5 | $C_6H_{11}$ | H | H | Br | 234-235 |
| 6 | $CH_3$ | $CH_3$ | H | OH | > 260 |
| 7 | $CH_3$ | H | $CH_3$ | Br | 195 |
| 8 | H | $C_2H_5$ | H | Br | 158-160 |
| 9 | H | $n-C_3H_7$ | H | Br | 171 |

### Beispiel 10

1-Methyl-1,2-dihydro-benzo[c][1,8]naphthyridin-3(4H)on

8,7 g (0.03 Mol) 1-Methyl-6-brom-1,2-dihydro-benzo-[c][1,8]naphthyridin-3(4H)-on werden in 750 ml Ethanol unter Zusatz von 2,4 g Natriumacetat und 1,2 g Palladium-C (10%) bei Raumtemperatur mit Wasserstoff (1 bar) behandelt. Nach Aufnahme der berechneten Menge Wasserstoff wird mit dem gleichen Volumen Ethanol oder Aceton verdünnt und erwärmt, um die ausgefallene Substanz in Lösung zu bringen. Der Katalysator wird abgetrennt und das Filtrat im Vakuum eingeengt. Der kristalline Niederschlag des 1-Methyl-1,2-dihydro-benzo[c][1,8]naphthyridin-3 (4H)-on wird abgesaugt und aus Methanol umkristallisiert. Fp. 261 - 263°C. Ausbeute: 5,8 g

Analog Beispiel 10 werden folgende Benzo-1,2-dihydro-naphthyridin-Verbindungen der Formel I (aromatischer C-Ring) aus den entsprechenden 6-Brom-Verbindungen hergestellt:

| Beispiel Nr. | $R^1$ | $R^2$ | $R^3$ | $R^4$ | Fp. °C |
|---|---|---|---|---|---|
| 11 | H | H | $CH_3$ | H | 225 |
| 12 | $C_6H_5$ | H | H | H | > 270 |
| 13 | H | H | COOEt | H | 237 |
| 14 | $CH_3$ | H | $CH_3$ | H | 205 |
| 15 | H | $C_2H_5$ | H | H | 214 |

## Beispiel 16

### 1-Methyl-1,2,7,8,9,10-hexahydro-benzo/c//1,8/naphthyridin-3(4H)-on

1,0 g 1-Methyl-1,2-dihydro-benzo/c//1,8/naphthyridin-3(4H)on werden in 50 ml Essigsäure gelöst und nach Zugabe von 0,3 g Rhodium/$Al_2O_3$ im Autoklaven bei 80°C und 100 bar hydriert. Nach Beendigung der Wasserstoffaufnahme wird der Katalysator abfiltriert und das Filtrat abgedampft. Nach Umkristallisation aus Ethanol erhält man 0,6 g 1-Methyl-1,2,7,8,9,10-hexahydro-benzo/c//1,8/naphthyridin-3(4H)-on. Fp. 265 - 266°C.

## Beispiel 17

1-Methyl-6-amino-1,2,7,8,9,10-hexahydro-benzo[c][1,8]-naphthyridin-3(4H)-on

Analog Beispiel 16 wurde aus 1-Methyl-6-benzyl-amino-1,2-dihydro-benzo[c][1,8]naphthyridin-3(4H)-on die Verbindung 1-Methyl-6-amino-1,2,7,8,9,10-hexahydro-benzo[c][1,8]naphthyridin-3(4H)-on erhalten. Fp. über 250°C.

## Beispiel 18

1-Methyl-6-methoxy-1,2-dihydro-benzo[c][1,8]naphthyridin-3(4H)-on

2,9 g (0.01 Mol) 1-Methyl-6-brom-1,2-dihydro-benzo-[c][1,8]naphthyridin-3(4H)-on werden mit 0,23 g (0.01 Mol) Natrium in 30 ml Methanol im Autoklaven 2 Stunden auf 180°C erhitzt. Die ausgeschiedenen Kristalle des 1-Methyl-6-methoxy-1,2-dihydro-benzo-[c][1,8]naphthyridin-3(4H)-ons werden aus Methanol umkristallisiert. Fp. 190 - 191°C.

## Beispiel 19

1-Methyl-6-n-propoxy-1,2-dihydro-benzo[c][1,8]-naphthyridin-3(4H)-on

Analog Beispiel 18 wurde aus 1-Methyl-6-brom-1,2-dihydro-benzo[c][1,8]naphthyridin-3(4H)-on mit n-Propanol 1-Methyl-6-n-propoxy-1,2-dihydro-benzo-[c][1,8]naphthyridin-3(4H)-on erhalten. Fp. 154°C.

## Beispiel 20

### 1-Methyl-6-dimethylamino-1,2-dihydro-benzo[c][1,8]-naphthyridin-3(4H)-on

2,0 g 1-Methyl-6-brom-1,2-dihydro-benzo[c][1,8]-naphthyridin-3(4H)-on werden in einer 50%igen äthanolischen Dimethylamin-Lösung im Autoklaven 4 Stunden auf 180°C erhitzt. Nach Eindampfen des Reaktionsgemisches wird der Rückstand 1-Methyl-6-dimethylamino-1,2-dihydro-benzo[c][1,8]naphthyridin-3(4H)-on aus Methanol umkristallisiert. Fp. 207 - 208°C. Ausbeute: 1,5 g.

In zu Beispiel 20 analoger Weise werden folgende erfindungsgemäße 1,2-Dihydro-benzonaphthyridin-Verbindungen der Formel I (aromatischer C-Ring) aus 1-Methyl-6-brom-1,2-dihydro-benzo[c][1,8]-naphthyridin-3(4H)-on mit den entsprechenden Aminen $H-R^4$, eingeschlossen die heterocyclischen Amine, Ammoniak oder Hydrazin hergestellt. Die Alkoxy- und Alkylthio-Verbindungen werden analog erhalten. Die Carboxamid-Verbindung wird durch partielle Verseifung der Cyanoverbindung hergestellt.

| Beispiel Nr. | $R^1$ | $R^2$ | $R^3$ | $R^4$ | Fp. °C |
|---|---|---|---|---|---|
| 21 | $CH_3$ | H | H | $NHCH_3$ | 190 |
| 22 | $CH_3$ | H | H | $N(C_2H_5)_2$ | 130-132 |
| 23 | $CH_3$ | H | H | $NHNH_2$ | 237 |
| 24 | $CH_3$ | H | H | $NH_2$ | > 250 |
| 25 | $CH_3$ | H | H | 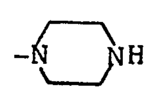 | 224-225 |
| 26 | $CH_3$ | H | H | 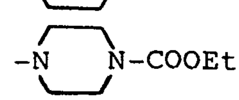 | 214 |

| Beispiel Nr. | $R^1$ | $R^2$ | $R^3$ | $R^4$ | Fp. °C |
|---|---|---|---|---|---|
| 27 | $CH_3$ | H | H | $NHCH_2C_6H_5$ | 176-177 |
| 28 | $CH_3$ | H | H | $N(n-C_3H_7)_2$ | Öl |
| 29 | $CH_3$ | H | H | $NH-n-C_4H_9$ | 178-179 |
| 30 | $CH_3$ | H | H | 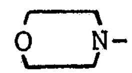 | 192-193 |
| 31 | $CH_3$ | H | H | 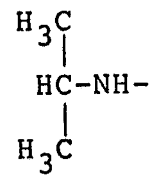 | 208-210 |
| 32 | $CH_3$ | H | H | 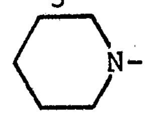 | 148 |
| 33 | $CH_3$ | H | H | 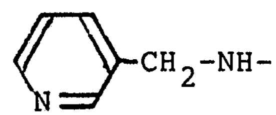 | 275 |
| 34 | $CH_3$ | H | H | 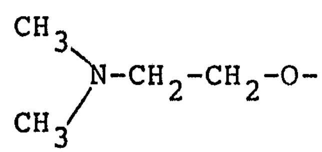 | 172-173 |

| Beispiel Nr. | $R^1$ | $R^2$ | $R^3$ | $R^4$ | Fp. °C |
|---|---|---|---|---|---|
| 35 | $CH_3$ | H | H | $CH_3-S-$ | 238-239 |
| 36 | $CH_3$ | H | H | $C_2H_5NH-$ | 223-224 |
| 37 | $CH_3$ | H | H | $N{\equiv}C-$ | 261 (z.) |
| 38 | $CH_3$ | H | H | 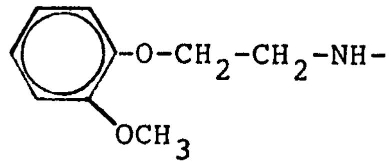 | 190-191 |
| 39 | $CH_3$ | H | H | 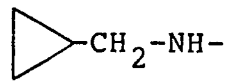 | 199-200 |
| 40 | $CH_3$ | H | H | 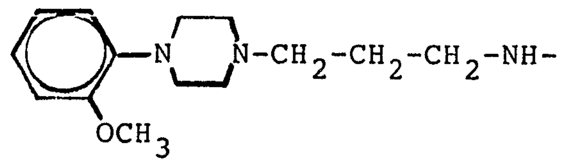 | 114-116 |
| 41 | $CH_3$ | H | H | 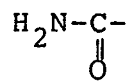 | 331-333 (z.) |
| 42 | $CH_3$ | H | H | 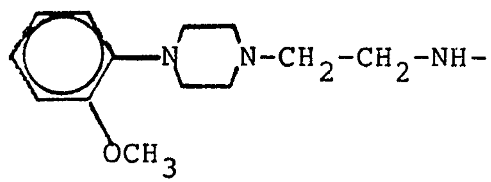 | 103-105 |
| 43 | $CH_3$ | H | H | 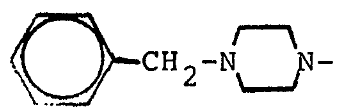 | 164-165 |

## Beispiel 44

### 1,2-Dihydro-3-oxo-4H-6-brom-benzo-[c][1,8]naphthyridin-2-carbonsäure

3,5 g 1,2-Dihydro-3-oxo-4H-6-brom-benzo[c][1,8]-naphthyridin-2-carbonsäureäthylester werden in 20 ml Ethanol mit 2N Natronlauge (20 ml) bei Raumtemperatur verseift. Nach Einengen und Ansäuern erhält man 2g der Carbonsäure. Fp. >250°C (Z.)

## Beispiel 45

### 3-Methyl-4-cyano-4-(2-cyano-phenyl)-buttersäuremethylester (Formel II)

Ein Gemisch von 7,1 g (0.05 Mol) 2-Cyano-benzylcyanid und 5,0 g (0.05 Mol) Crotonsäuremethylester wird mit wenigen Tropfen einer Natriummethylat-Lösung versetzt und nach Abklingen der Reaktion mit Essigsäure neutralisiert. Man nimmt das Reaktionsgemisch in Methylenchlorid auf, schüttelt mit Wasser gegen und erhält nach Abdampfen des Lösungsmittels einen öligen Rückstand von 3-Methyl-4-cyano-4-(2-cyano-phenyl)-buttersäuremethylester. Die Strukturaufklärung wurde durch NMR-Spektroskopie vorgenommen:

NMR ($CDCl_3$): $\delta$ 3.6 u. 3.7 (3H,s,$OCH_3$), 1.15 (3H,m,$CH_3$), 4.33 u. 4.48 (1H,d,CHCN), 2.1 - 3.0 (3H,m,-<u>CH</u>($CH_3$) + $CH_2CO$)

Analog wurden die folgenden Additionsprodukte der Formel II aus 2-Cyano-benzylcyanid und den entsprechenden Propensäureestern synthetisiert:

2-Methyl-4-cyano-4-(2-cyano-phenyl)-buttersäuremethylester
3-Phenyl-4-cyano-4-(2-cyano-phenyl)-buttersäuremethylester
3-Cyclohexyl-4-cyano-4-(2-cyano-phenyl)-buttersäuremethylester
2,3-Dimethyl-4-cyano-4-(2-cyano-phenyl)-buttersäuremethylester
3,3-Dimethyl-4-cyano-4-(2-cyano-phenyl)-buttersäuremethylester
3-Ethyl-4-cyano-4-(2-cyano-phenyl)-buttersäuremethylester
3-Propyl-4-cyano-4-(2-cyano-phenyl)-buttersäuremethylester
2-Cyano-2-(2-cyano-phenyl)-äthyl-malonsäurediäthylester

Beispiel 46

Herstellung von Tabletten

Tabletten, die die im folgenden genannten Bestandteile enthalten, lassen sich in bekannter Weise herstellen. Sie können zur Behandlung von den vorstehend genannten Krankheiten, insbesondere der Herzinsuffizienz, in einer Dosierung von einer Tablette einmal bis zweimal täglich angewendet werden.

| | |
|---|---|
| 1-Methyl-6-diethylamino-1,2-dihydro-benzo[c][1,8]naphthyridin-3(4H)-on | 15 mg |
| Lactose | 75 mg |
| Maisstärke | 10 mg |
| Mikrokristalline Cellulose | 8 mg |
| Polyvinylpyrrolidon | 1 mg |
| Magnesiumstearat | 0,5 mg |
| hochdisperses Siliziumdioxid | 0,5 mg |

Patentansprüche

1. Benzo/c//1,8/naphthyridine der allgemeinen Formel I,

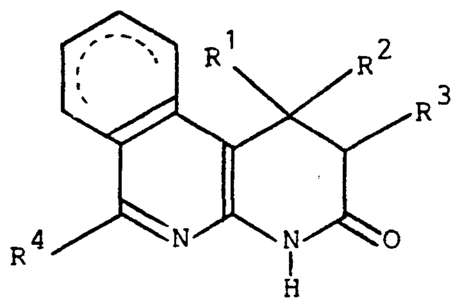

(I)

worin $R^1$ Wasserstoff, eine Alkylgruppe mit 1 bis 6 Kohlenstoffatomen, die Phenylgruppe oder eine substituierte Phenylgruppe, eine Cycloalkylgruppe, eine Carboxylgruppe, Alkoxycarbonylgruppe, Carboxamidgruppe oder Cyanogruppe,

$R^2$ Wasserstoff oder eine Alkylgruppe mit 1 bis 4 Kohlenstoffatomen,

$R^3$ Wasserstoff, eine Alkylgruppe mit 1 bis 4 Kohlenstoffatomen, eine Carboxylgruppe, Alkoxycarbonylgruppe, Carboxamidgruppe oder Cyanogruppe und

$R^4$ Wasserstoff, Halogen, die Cyanogruppe, Hydroxylgruppe, Carboxamidgruppe, eine Alkylthiogruppe oder Alkoxygruppe mit jeweils 1 bis 6 Kohlenstoffatomen, die gegebenenfalls durch substituierte Aminogruppen substituiert sein können, eine Aminogruppe oder substituierte Aminogruppe, eine Pyrrolidino-, Piperidino-, Hydrazino-, Morpholino-, Piperazino- oder substituierte Piperazinogruppe,

bedeutet, wobei, wie durch punktierte Bindungen angegeben, Ring C des Benzonaphthyridingerüstes auch in partiell hydrierter Form vorliegen kann und Alkyle jeweils geradkettig oder verzweigt sein können, sowie ihre tautomeren Formen und ihre Salze sowie Säureadditionssalze, zur Anwendung als therapeutische Wirkstoffe.

2. Benzo/c//1,8/naphthyridine der allgemeinen Formel I,

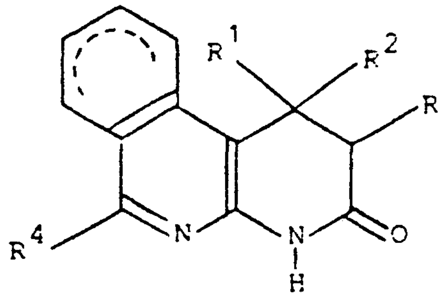

(I)

worin $R^1$ Wasserstoff,
eine Alkylgruppe mit 1 bis 6 Kohlenstoffatomen,
die Phenylgruppe oder eine substituierte Phenylgruppe,
eine Cycloalkylgruppe, eine Carboxylgruppe, Alkoxycarbonylgruppe, Carboxamidgruppe oder Cyanogruppe,

$R^2$ Wasserstoff oder eine Alkylgruppe mit 1 bis 4 Kohlenstoffatomen,

$R^3$ Wasserstoff, eine Alkylgruppe mit 1 bis 4 Kohlenstoffatomen, eine Carboxylgruppe, Alkoxycarbonylgruppe, Carboxamidgruppe oder Cyanogruppe und

$R^4$ Wasserstoff, Halogen, die Cyanogruppe, Hydroxylgruppe, Carboxamidgruppe, eine Alkylthiogruppe oder Alkoxygruppe mit jeweils 1 bis 6 Kohlenstoffatomen, die gegebenenfalls durch substituierte Aminogruppen substituiert sein können, eine Aminogruppe oder

substituierte Aminogruppe, eine Pyrrolidino-, Piperidino-, Hydrazino-, Morpholino-, Piperazino- oder substituierte Piperazinogruppe,

BAD ORIGINAL

bedeutet, wobei, wie durch punktierte Bindungen angegeben, Ring C des Benzonaphthyridingerüstes auch in partiell hydrierter Form vorliegen kann und Alkyle jeweils geradkettig oder verzweigt sein können, sowie ihre tautomeren Formen und ihre Salze sowie Säureadditionssalze, mit Ausnahme von 1,2-Dihydro-benzo[c][1,8]naphthyridin -3(4H)-on und 6-Brom-1,2-dihydrobenzo[c][1,8]naphthyridin-3(4H)-on.

3. Benzo[c][1,8]naphthyridine gemäß Anspruch 1 oder 2, dadurch gekennzeichnet, daß die substituierten Aminogruppen $R^4$ mono- oder disubstituierte Aminogruppen-$NR^5R^6$ bedeuten, worin $R^5$ und $R^6$, die gleich oder verschieden sein können, Wasserstoff, Arylalkylgruppen, verzweigte oder unverzweigte Alkylgruppen mit jeweils 1 bis 6 Kohlenstoffatomen sind, die gegebenenfalls durch Alkoxy-, Aryloxy- oder substituierte Aryloxygruppen, Pyridinylgruppen, Cycloalkylgruppen oder substituierte Aminogruppen substituiert sein können.

4. Verfahren zur Herstellung der Verbindungen gemäß Anspruch 1, dadurch gekennzeichnet, daß man Verbindungen der Formel II,

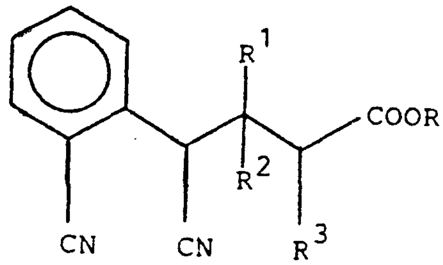

(II)

in der $R^1$, $R^2$ und $R^3$ die oben angegebene Bedeutung haben und R eine niedere Alkylgruppe bedeutet, mit einer Halogenwasserstoffsäure in an sich bekannter Weise zu Verbindungen der Formel I cyclisiert, in der $R^4$ Halogen bedeutet und gegebenenfalls diese 6-Halogenverbindungen

a) zur Einführung des Restes $R^4$ = Wasserstoff hydriert,

b) zur Herstellung anderer 6-Halogenverbindungen mit entsprechenden Halogeniden umsetzt,

c) zur Herstellung von 6-Cyanoverbindungen mit Cyaniden,

d) zur Herstellung von 6-Hydroxyverbindungen mit Basen,

e) zur Herstellung von 6-Alkoxyverbindungen mit den entsprechenden Alkoxylaten,

f) zur Herstellung der 6-Aminoverbindungen mit Ammoniak,

g) zur Herstellung der Verbindungen, der Formel I, in der $R^4$ eine substituierte Aminogruppe bedeutet mit entsprechenden Aminen der Formel $H-R^4$,

h) zur Herstellung von 6-Alkylthioverbindungen mit den entsprechenden Mercaptanen umsetzt und

i) zur Herstellung der 6-Carboxamide die Cyanogruppe partiell verseift,

und gegebenenfalls Verbindungen der Formel I, in der Ring C des Benzonaphthyridingerüstes aromatisch ist, zur Herstellung der im C-Ring partiell hydrierten Verbindungen der Formel I hydriert.

5. Verfahren zur Herstellung der Verbindungen der Formel I gemäß Anspruch 1, in der $R^1$ und/oder $R^3$ die Carboxyl-, Carboxamid- oder Cyanogruppe bedeuten, dadurch gekennzeichnet, daß man die Carbonsäureestergruppen $R^1$ und/oder $R^3$ in an sich bekannter Weise zur Herstellung der Carbonsäuren spaltet, zur Herstellung der Amide mit Ammoniak umsetzt und zur Herstellung der Cyanoverbindungen die Amide dehydratisiert.

6. Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält.

7. Verbindungen der Formel I gemäß Anspruch 1 zur Behandlung von Herzinsuffizienz, Angina pectoris und Hypertonie.

Europäisches Patentamt

**EUROPÄISCHER RECHERCHENBERICHT**

0167045

Nummer der Anmeldung

EP 85 10 7403

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | GB-A-1 397 869 (MERCK)<br>* Patentanspruch 1; Seite 2, Zeilen 12-15 *<br>----- | 1,6 | C 07 D 471/04<br>A 61 K 31/435//<br>(C 07 D 471/04<br>C 07 D 221:00<br>C 07 D 221:00 ) |
| | | | **RECHERCHIERTE SACHGEBIETE (Int. Cl.4)** |
| | | | C 07 D 471/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 27-09-1985 | ALFARO I. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82